# EUROPEAN PATENT APPLICATION

(11) **EP 3 047 841 A1**
(43) Date of publication of application: **27.07.2016**
(21) Application number: 16152065.5
(22) Date of filing: 20.01.2016
(51) Int. Cl.: A61K 8/34, A61K 8/41, A61Q 5/10

(54) **STABILITY IMPROVER OF AROMATIC COMPOUND AND METHOD FOR IMPROVING STABILITY OF AROMATIC COMPOUND**

(30) Priority: 22.01.2015 JP 2015010748
(71) Applicant: Hoyu Co., Ltd., Nagoya-shi Aichi, 461-8650 (JP)
(72) Inventor: KOBAYASHI, Yosuke, Nagakute-shi,, Aichi 480-1136 (JP)
(74) Representative: TBK

(57) **Abstract**

To provide a stability improver of an aromatic compound, which is capable of improving stability of the aromatic compound, and a method for improving stability of an aromatic compound.

The stability improver of an aromatic compound of the present invention includes (A) at least one selected from resorcin and its derivatives, and salts thereof. The aromatic compound may be at least one selected from an aromatic amine, an aromatic nitro compound, a phenol compound, an aromatic azo compound, and a fused ring compound.

## Description

### [Technical Field]

The present invention relates to a stability improver of an aromatic compound and a method for improving stability of an aromatic compound.

### [Background Art]

For example, aromatic compounds are known as a group of cyclic unsaturated organic compounds, such as benzene. As the aromatic compounds, benzene-based aromatic compounds, heteroaromatic compounds, and non-benzene-based aromatic compounds are known and widely utilized in the field of chemical industry and the like. Though a lot of aromatic compounds are relatively stable, there is a concern that the stability thereof is lowered in a physical or chemical stress circumstance or in a long-term preservation environment.

There has hitherto been known a compound capable of improving the stability of an aromatic compound as disclosed in Patent Literature 1. Patent Literature 1 discloses an antioxidant, such as ascorbic acid, a sulfite, etc., as the compound capable of improving stability of a dye through a combined use with a nitro dye that is the aromatic compound.

### [Citation List]

### [Patent Literature]

Patent Literature 1: JP-A-2010-105998
Patent Literature 2: JP-A-2003-103398
Patent Literature 3: JP-A-2004-137491

### [Summary of Invention]

### [Technical Problem]

However, in the antioxidant which has hitherto been used, such as ascorbic acid, etc., there was involved such a problem that its effect for improving the stability of an aromatic compound is still insufficient.

Now, resorcin is known as a compound having a structure in which two hydroxyl groups are present at the meta position of a benzene ring. As disclosed in Patent Literatures 2 and 3, resorcin has hitherto been utilized as an antiseptic or the like, in addition to a raw material of adhesives and a raw material of flame retardants for resin.

The present invention is made on the basis of finding that resorcin or the like is able to improve stability of an aromatic compound. An object of the present invention is to provide a stability improver of an aromatic compound, which is capable of improving stability of the aromatic compound, and a method for improving stability of an aromatic compound.

### [Solution to Problem]

A numerical value expressing a content of each component in terms of % by mass is a numerical value in a dosage form including a solubilizer, such as water, etc. In order to attain the above-described object, a first aspect of the present invention is concerned with a stability improver of an aromatic compound comprising (A) at least one selected from resorcin and its derivatives, and salts thereof.

The above-described aromatic compound may be at least one selected from an aromatic amine, an aromatic nitro compound, a phenol compound, an aromatic azo compound, and a fused ring compound. The above-described aromatic compound may also be at least one selected from compounds represented by the following general formulae (1) to (3). 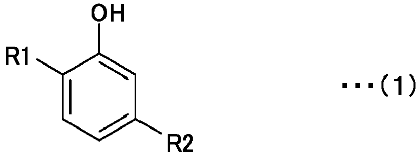 (R1 represents a hydrogen atom or a methyl group; and R2 represents an amino group or a substituted amino group.) 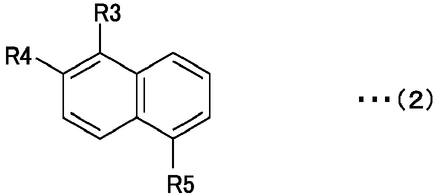 (Each of R3 to R5 represents a hydrogen atom or a hydroxyl group, and at least one of R3 to R5 represents a hydroxyl group.) 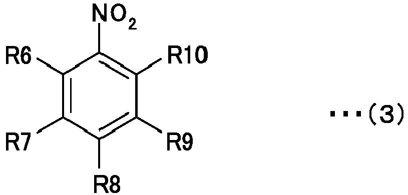 (Each of R6 to R10 represents a hydrogen atom, an amino group, a substituted amino group, a nitro group, or a hydroxyl group.)

The above-described aromatic compound may also be at least one selected from m-aminophenol, 5-amino-o-cresol, α-naphthol, 5-(2-hydroxyethylamino)-2-methylphenol, 1,5-dihydroxynaphthalene, and a nitro dye.

Another embodiment of the present invention is concerned with a method for improving stability of an aromatic compound, which comprises mixing an aromatic compound with at least one compound selected from resorcin and its derivatives, and salts thereof in a solution.

### [Advantageous Effects of Invention]

According to the present invention, stability of an aromatic compound can be improved.

### [Description of Embodiments]

An embodiment embodying the stability improver of an aromatic compound of the present invention is hereunder described.

The stability improver of the present embodiment comprises (A) at least one selected from resorcin and its derivatives, and salts thereof (hereinafter referred to as "resorcin or the like"). Examples of the derivative of resorcin include an alkylated resorcin, a halogenated resorcin, and the like. Specific examples of the alkylated resorcin include 2-methylresorcin and the like. Specific examples of the halogenated resorcin include 4-chlororesorcin, 2-chlororesorcin, and the like. Among those resorcins or the like, only one may be contained solely, or a combination of two or more thereof may also be contained.

Though the aromatic compound to which the resorcin or the like of the present embodiment is applied is not particularly limited, any compounds can be applied so long as they are classified into a benzene-based aromatic compound, a heteroaromatic compound, or a non-benzene-based aromatic compound. It is to be noted that it should be construed that the resorcin or the like is not included in the aromatic compound.

Examples of the benzene-based aromatic compound include an aromatic amine, an aromatic nitro compound, a phenol compound, an aromatic azo compound, an aromatic hydrocarbon, such as toluene, etc., an aromatic carboxylic acid, such as benzoic acid, etc., and the like. In addition, an aromatic polycyclic compound and a fused ring compound are also classified as the benzene-based aromatic compound. Examples of the aromatic polycyclic compound include biphenyl, triphenylmethane, phenolphthalein, and the like. Examples of the fused ring compound include an acene, phenanthrene, chrysene, triphenylene, tetraphene, pyrene, picene, pentaphene, perylene, helicene, coronene, and the like.

Examples of the heteroaromatic compound include furan, thiophene, pyrrole, imidazole, and the like. Examples of the non-benzene-based aromatic compound include annulene, azulene, cyclopentadienyl anion, cycloheptatrienyl cation, tropone, metallocene, acepleiadylene, and the like.

Among those specific examples of the aromatic compound, only one may be contained solely, or a combination of two or more thereof may also be contained.

The stability improver of the present embodiment is preferably applied to an aromatic amine, an aromatic nitro compound, a phenol compound, an aromatic azo compound, and a fused ring compound among these aromatic compounds. The stability improver of the present embodiment is able to more improve the stability against these compounds.

Examples of the phenol compound include a compound represented by the following general formula (1). 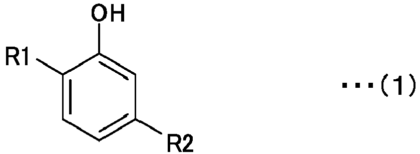
(R1 represents a hydrogen atom or a methyl group; and
R2 represents an amino group or a substituted amino group.)

Specific examples of the compound represented by the general formula (1) include m-aminophenol, 5-amino-o-creasol, 5-(2-hydroxyethylamino)-2-methylphenol, and the like.

Examples of the fused ring compound include the above-described acene. Examples of the acene include a compound represented by the following general formula (2). 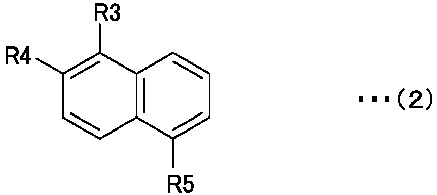 (Each of R3 to R5 represents a hydrogen atom or a hydroxyl group, and at least one of R3 to R5 represents a hydroxyl group.)

Specific examples of the compound represented by the general formula (2) include α-naphthol, 1,5-dihydroxynaphthalene, and the like.

Examples of the aromatic nitro compound or aromatic azo compound include a compound or nitro dye represented by the following general formula (3). 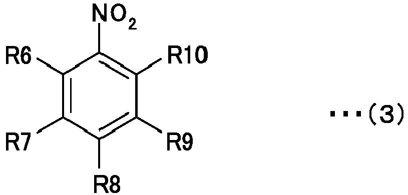 (Each of R6 to R10 represents a hydrogen atom, an amino group, a substituted amino group, a nitro group, or a hydroxyl group.)

Specific examples of the nitro dye include 4-nitro-o-phenylenediamine, 2-nitro-p-phenylenediamine, 2-amino-4-nitrophenol, 2-amino-5-nitrophenol, picramic acid, picric acid, and salts thereof, as well as HC Blue No. 2, HC Blue No. 4, HC Blue No. 5, HC Blue No. 6, HC Blue No. 8, HC Blue No. 9, HC Blue No. 10, HC Blue No. 11, HC Blue No. 12, HC Blue No. 13, HC Blue No. 14, HC Brown No. 1, HC Brown No. 2, HC Green No. 1, HC Orange No. 1, HC Orange No. 2, HC Orange No. 3, HC Orange No. 5, HC Red No. 1, HC Red No. 3, HC Red No. 7, HC Red No. 8, HC Red No. 9, HC Red No. 10, HC Red No. 11, HC Red No. 13, HC Red No. 14, HC Violet No. 1, HC Violet No. 2, HC Yellow No. 4, HC Yellow No. 5, HC Yellow No. 6, HC Yellow No. 7, HC Yellow No. 8, HC Yellow No. 9, HC Yellow No. 10, HC Yellow No. 11, HC Yellow No. 12, HC Yellow No. 13, HC Yellow No. 14, HC Yellow No. 15, and the like. Among those specific examples of the nitro dye, only one may be contained solely, or a combination of two or more thereof may also be contained. Among those specific examples of the nitro dye, 4-nitro-o-phenylenediamine, 2-nitro-p-phenylenediamine, HC Blue No. 2, and HC Yellow No. 4 are preferably applied from the viewpoint of improving the stability of color tone.

Next, a method for using the stability improver (method for improving stability of an aromatic compound) of the present embodiment is described.

The stability improver of the present embodiment is used upon being mixed with the aromatic compound. The mixing of the stability improver of the present embodiment with the aromatic compound may be made in any form of a solution (liquid) and a solid. The form of a solution is prepared by using a solvent, and it may be any of a gel form, a foamy form, and a cream form. In addition, the solution may also be a form, such as a dispersion liquid, an emulsion, etc. The stability improver of the present embodiment is preferably used for the purpose of improving the stability of the aromatic compound in the solution.

As the solvent, a known solvent can be properly chosen while taking into consideration solubility of the aromatic compound, an application, or the like. For example, in the case where the compounds represented by the foregoing general formulae (1) to (3) are applied as the aromatic compound, water and an organic solvent are preferably used. Specific examples of the organic solvent include ethanol, n-propanol, isopropanol, methyl cellosolve, ethyl cellosolve, methyl carbitol, ethyl carbitol, benzyl alcohol, phenethyl alcohol, γ-phenylpropyl alcohol, cinnamic alcohol, anise alcohol, p-methylbenzyl alcohol, α-dimethylphenethyl alcohol, α-phenylethanol, phenoxyethanol, phenoxyisopropanol, 2-benzyloxyethanol, an N-alkylpyrrolidone, an alkylene carbonate, and an alkyl ether. Among those solvents, only one may be contained solely, or a combination of two or more thereof may also be contained.

Though a mass ratio of a content of the aromatic compound to a content of the resorcin or the like at the time of use is properly set, a lower limit thereof is preferably 0.001 or more, more preferably 0.01 or more, and still more preferably 0.02 or more. When such a mass ratio is 0.001 or more, the stability of the aromatic compound can be more improved. Though an upper limit of such a mass ratio is properly set, it is preferably 100 or less, more preferably 80 or less, and still more preferably 60 or less. When such a mass ratio is 100 or less, revelation of actions which the resorcin or the like has, other than the stability of the aromatic compound, can be more suppressed.

Next, the action of the stability improver of the present embodiment is described.

The stability improver of the present embodiment is used upon being mixed with an aromatic compound, whereby the stability of the aromatic compound can be enhanced. In aromatic compounds, the stability is lowered under influences by various physical or chemical stresses, for example, heat, light, ultraviolet rays, radiations, oxidation, acids or alkalis, or the copresence of other compound, or in a long-term preservation environment, or the like.

The stability improver of the present embodiment is used for improving the stability of the aromatic compound especially in the copresence of a carbonate or an alkali agent. In aromatic compounds, especially the compounds represented by the foregoing general formulae (1) to (3), there is a concern that the stability thereof is more lowered in the copresence of a carbonate or an alkali agent. According to the stability improver of the present embodiment, a lowering of the stability of the aromatic compound to be caused in the copresence of a carbonate or an alkali agent can be more suppressed.

The carbonate is not particularly limited, and examples thereof include known carbonates, for example, sodium carbonate, sodium hydrogencarbonate, potassium carbonate, potassium hydrogencarbonate, lithium carbonate, calcium carbonate, magnesium carbonate, barium carbonate, guanidine carbonate, guanidine hydrogencarbonate, ammonium carbonate, and ammonium hydrogencarbonate. Among those carbonates, only one may be contained solely, or a combination of two or more thereof may also be contained.

Examples of the alkali agent other than the above-described carbonate include ammonia, an alkanolamine, a silicate, a metasilicate, a sulfate, a chloride, a phosphate, an organic amine, and a basic amino acid. Specific examples of the alkanolamine include monoethanolamine and triethanolamine. Specific examples of the silicate include sodium silicate and potassium silicate. Specific examples of the metasilicate include sodium metasilicate and potassium metasilicate. Specific examples of the sulfate include ammonium sulfate. Specific examples of the chloride include ammonium chloride. Specific examples of the phosphate include primary ammonium phosphate and secondary ammonium phosphate. Examples of the organic amine include 2-amino-2-methyl-1-propanol (AMP), 2-amino-2-methyl-1,3-propanediol, and guanidine. Specific examples of the basic amino acid include arginine and lysine. Among those alkali agents other than the carbonate, only one may be contained solely, or a combination of two or more thereof may also be contained.

The stability improver according to the present embodiment brings about the following advantages.
(1) The stability improver of an aromatic compound of the present embodiment contains resorcin or the like. Accordingly, the stability improver of the present embodiment can improve the stability of an aromatic compound. In particular, the stability improver of the present embodiment can more improve the stability of the aromatic compound under influences by various physical or chemical stresses, for example, heat, light, ultraviolet rays, radiations, oxidation, acids or alkalis, or the copresence of other compound, or in a long-term preservation environment, or the like. The stability improver of the present embodiment is more excellent in the stability improving action per unit mass than known antioxidants, for example, ascorbic acid.
(2) In particular, the stability improver of the present embodiment is preferably applied to an aromatic amine, an aromatic nitro compound, a phenol compound, an aromatic azo compound, and a fused ring compound as the aromatic compound. The stability improver of the present embodiment can more improve the stability against those compounds.
(3) In particular, the stability improver of the present embodiment is used for improving the stability of an aromatic compound in the copresence of a carbonate or an alkali agent. The stability improver of the present embodiment is able to suppress a lowering of the stability of an aromatic compound in the copresence of a carbonate or an alkali agent.

The above-described embodiment may be modified in the following manners.
- In the above-described embodiment, the stability improver may also be used in combination with a known antioxidant. According to such a constitution, it is expected that the stability of an aromatic compound can be more improved. It is to be noted that examples of the known antioxidant include ascorbic acid, α-tocopherol, a thiol compound, BHA, glucose, flavonoid, SOD, peroxidase, and the like.
- The stability improver of an aromatic compound of the present embodiment may be constituted of only resorcin or the like, or may be compounded with various known additives within the range where the effects of the present invention are not impaired. The additive is not particularly limited, and examples thereof include a component contributing to an improvement of the stability of an aromatic compound, such as a pH adjustor, a chelating agent, etc., a solvent, and the like.
- The stability improver of an aromatic compound of the present embodiment can be used upon being added and mixed in an application of the aromatic compound for the purpose of every sort. The application for which the aromatic compound is used is not particularly limited, and examples thereof include applications in the fields of various chemical industries and the like. More specifically, examples thereof include fields of paints, dyes, pigments, perfumes, cosmetics, medicines, agricultural chemicals, food additives, surfactants, and the like.

For example, in the case where the aromatic compound is applied in the field of dyes, there is exemplified a constitution of a first agent for oxidation hair dye containing an oxidation dye or a nitro dye as the aromatic compound. For the purpose of improving stability of the dye, the resorcin or the like can be used upon being added and mixed in the first agent for oxidation hair dye. In such a constitution, though an upper limit of a content of the resorcin or the like in the first agent for oxidation hair dye is properly set, it is preferably 0.2% by mass or less, more preferably 0.15% by mass or less, and still more preferably 0.1% by mass or less. When the content of the resorcin or the like is 0.2% by mass or less, revelation of a color tone originated from the resorcin or the like can be more suppressed.

### [Examples]

Next, the above-described embodiments are more specifically described by reference to Examples and Comparative Examples. It is to be noted that the present invention is not limited to the constitutions described in the Examples.

An aromatic compound-containing solution of each of the Examples and the Comparative Examples, the solution containing each of components shown in Tables 1 and 2 and being alkaline, was prepared. Compounds enumerated in Tables 1 and 2, specifically oxidation dyes or nitro dyes were used, respectively as the aromatic compound. Numerical values in the columns expressing the respective components in Tables 1 and 2 show contents of components in the instant columns, and units thereof are % by mass. The resulting aromatic compound-containing solution of each of the Examples and the Comparative Examples was preserved in a thermostat at 45°C for one month. By using the aromatic compound-containing solution of each of the Examples and the Comparative Examples, which had been preserved for a prescribed period of time, the stability of the aromatic compound of each of the Examples and the Comparative Examples was evaluated by a method as described below. It is to be noted that since easiness of oxidation with oxygen in air during the long-term preservation differs depending upon each compound, a small amount of ascorbic acid is used in combination for the purposes of suppressing deterioration of the aromatic compound to be caused due to oxidation and undergoing the accurate evaluation. In addition, the expression (A) in the "Component" column in the tables expresses the compound corresponding to the component (A) as described in the claims of the present application. The expression of (a) expresses a control compound of the component (A) as described in the claims of the present application.

### <Stability>

First of all, the aromatic compound-containing solution of each of the Examples and the Comparative Examples, which had been preserved for a prescribed period of time, was compounded with p-phenylenediamine as a dye intermediate in an amount of 0.02% by mass, thereby preparing a first agent for oxidation hair dye composition. Subsequently, a second agent for oxidation hair dye composition containing each of components shown in Table 3 was prepared. A numerical value in the column expressing each component in Table 3 shows a content of the component in the instant column, and a unit thereof is % by mass. Then, the first agent and the second agent were mixed in a mass ratio of 1/1 to prepare an oxidation hair dye composition. The resulting oxidation hair dye composition was applied to a bundle of human black hair (15 cm, available from Beaulax Co., Ltd.) (hereafter simply referred to as "hair bundle") by using a brush and then allowed to stand at room temperature (25°C) for 30 minutes. Subsequently, the oxidation hair dye composition attached onto the hair bundle was washed off with water, and thereafter, the hair bundle was applied with a shampoo ("Bigen Treatment Shampoo", available from Hoyu Co., Ltd.) twice and a rinse ("Bigen Treatment Rinse", available from Hoyu Co., Ltd.) once. Subsequently, the hair bundle was dried with warm air and then allowed to stand for one day. The hair bundle to which the hair dyeing treatment had been applied was evaluated with respect to a change of the color tone according to the following method.

The aromatic compound-containing solution of each of the Examples and the Comparative Examples, which had not been subjected to a preservation treatment, was used as a control and subjected to a dyeing treatment by adopting the same method as described above, thereby obtaining each hair bundle. With respect to each of the resulting hair bundles, the presence or absence of a change of color tone (lightness and chroma) to be caused due to the presence or absence of the preservation treatment was visually observed under a standard light source by ten expert panelists, and the evaluation was made according to the following criteria.

The results were marked with three grades in such a manner that the case where a change was not substantially observed in the hair dyeing results as compared with the control is given a score 3; the case where a change was slightly observed in the hair dyeing results as compared with the control is given a score 2; and the case where a change was observed in the hair dyeing results as compared with the control is given a score 1. With respect to the marking results by each of the expert panelists, an average value was calculated. The evaluation results are designated in such a manner that the case where the average value was 2.6 or more is defined as "A"; the case where the average value was 1.6 or more and less than 2.6 is defined as "B"; and the case where the average value was less than 1.6 is defined as "C". The results are shown in Tables 1 and 2.

**Table 1**

| | | Example 1 | Example 2 | Example 3 | Example 4 | Example 5 | Example 6 | Example 7 | Example 8 | Example 9 | Example 10 | Example 11 |
|---|---|---|---|---|---|---|---|---|---|---|---|---|
| (A) | Resorcin | 0.05 | 0.05 | 0.05 | 0.05 | 0.05 | 0.05 | 0.2 | 0.5 | 2 | 0.05 | 0.05 |
| | L-Ascorbic acid | 0.3 | 0.3 | 0.3 | 0.3 | 0.3 | 0.3 | 0.3 | 0.3 | 0.3 | 0.3 | 0.3 |
| Aromatic compound | m-Aminophenol | 0.03 | | | | | | 0.03 | 0.03 | 0.03 | 2 | 0.03 |
| | 5-Amino-o-cresol | | 0.03 | | | | | | | | | |
| | α-Naphthol | | | 0.03 | | | | | | | | |
| | 1,5-Dihydroxynaphthalene | | | | 0.03 | | | | | | | |
| | 5-(2-Hydroxyethylamino)-2-methylphenol | | | | | 0.03 | | | | | | |
| | 4-Nitro-o-phenylenediamine | | | | | | 0.03 | | | | | |
| | 28% Ammonia water | 5.5 | 5.5 | 5.5 | 5.5 | 5.5 | 5.5 | 5.5 | 5.5 | 5.5 | 5.5 | 5.5 |
| | Ammonium hydrogencarbonate | 4 | 4 | 4 | 4 | 4 | 4 | 4 | 4 | 4 | 4 | |
| | Monoethanolamine | | | | | | | | | | | 4 |
| | Purified water | Balance | Balance | Balance | Balance | Balance | Balance | Balance | Balance | Balance | Balance | Balance |
| | Whole amount | 100 | 100 | 100 | 100 | 100 | 100 | 100 | 100 | 100 | 100 | 100 |
| | | | | | | | | | | | | |
| | Mass ratio of (aromatic compound)/(resorcin) | 0.6 | 0.6 | 0.6 | 0.6 | 0.6 | 0.6 | 0.15 | 0.06 | 0.02 | 40 | 0.6 |
| | | | | | | | | | | | | |
| Evaluation | Stability | A | A | A | A | A | A | A | A | A | A | A |

**Table 2**

| | | Comparative Example 1 | Comparative Example 2 | Comparative Example 3 | Comparative Example 4 | Comparative Example 5 | Comparative Example 6 | Comparative Example 7 | Comparative Example 8 | Comparative Example 9 | Comparative Example 10 | Comparative Example 11 | Comparative Example 12 | Comparative Example 13 |
|---|---|---|---|---|---|---|---|---|---|---|---|---|---|---|
| | Salicylic acid | 0.05 | | | | | | | | | | | | |
| | Sodium benzoate | | 0.05 | | | | | | | | | | | |
| | Methyl paraoxybenzoate (methyl paraben) | | | 0.05 | | | | | | | | | | |
| | Ethyl p-aminobenzoate | | | | 0.05 | | | | | | | | | |
| (a) | Xylose | | | | | 0.05 | | | | | | | | |
| | Glucose | | | | | | 0.05 | | | | | | | |
| | Citric acid | | | | | | | 0.05 | | | | | | |
| | Tartaric acid | | | | | | | | 0.05 | | | | | |
| | dl-α-Tocopherol | | | | | | | | | 0.05 | | | | |
| | L-Cysteine hydrochloride | | | | | | | | | | 0.05 | | | |
| | Sodium sulfate | | | | | | | | | | | 0.05 | | |
| | L-Ascorbic acid | | | | | | | | | | | | 0.05 | |
| | Sodium sulfite | | | | | | | | | | | | | 0.05 |
| | L-Ascorbic acid | 0.3 | 0.3 | 0.3 | 0.3 | 0.3 | 0.3 | 0.3 | 0.3 | 0.3 | 0.3 | 0.3 | 0.3 | 0.3 |
| Aromatic compound | m-Aminophenol | 0.03 | 0.03 | 0.03 | 0.03 | 0.03 | 0.03 | 0.03 | 0.03 | 0.03 | 0.03 | 0.03 | 0.03 | 0.03 |
| | 28% Ammonia water | 5.5 | 5.5 | 5.5 | 5.5 | 5.5 | 5.5 | 5.5 | 5.5 | 5.5 | 5.5 | 5.5 | 5.5 | 5.5 |
| | Ammonium hydrogencarbonate | 4 | 4 | 4 | 4 | 4 | 4 | 4 | 4 | 4 | 4 | 4 | 4 | 4 |
| | Purified water | Balance | Balance | Balance | Balance | Balance | Balance | Balance | Balance | Balance | Balance | Balance | Balance | Balance |
| | Whole amount | 100 | 100 | 100 | 100 | 100 | 100 | 100 | 100 | 100 | 100 | 100 | 100 | 100 |
| | | | | | | | | | | | | | | |
| | Mass ratio of (aromatic compound)/(resorcin) | - | - | - | - | - | - | - | - | - | - | - | - | - |
| | | | | | | | | | | | | | | |
| Evaluation | Stability | C | C | C | C | C | C | C | C | C | C | C | C | C |

**Table 3**

| <Second agent> | |
|---|---|
| 35% Hydrogen peroxide | 15.7 |
| Hvdroxvethanediphosphonic acid | 0.2 |
| Tetrasodium hydroxyethanediphosphonate | 0.3 |
| Phenoxyethanol | 0.2 |
| Microcrystalline wax | 5.0 |
| Cetanol | 4.0 |
| Stearyl alcohol | 1.0 |
| POE(30) cetyl ether | 1.0 |
| POE(2) cetyl ether | 0.5 |
| Stearyl trimethyl ammonium chloride | 4.0 |
| Vaseline | 2.0 |
| Purified water | Balance |
| Whole amount | 100 |

As shown in Tables 1 and 2, it was understood that in the aromatic compound-containing solution of each of the Examples, the evaluation in the stability is high as compared with that of each of the Comparative Examples.

As shown in Tables 1 and 2, it was understood that in each of the Comparative Examples using various stabilizers other than resorcin, the evaluation in the stability is low as compared with that of each of the Examples.

Next, technical ideas that can be grasped from the above-described embodiments and the modifications are described additionally below along with effects thereof.
(a) The method for improving stability of an aromatic compound, wherein a mass ratio of a content of the aromatic compound to a content of (A) at least one selected from resorcin and its derivatives, and salts thereof is 0.001 to 100. According to such a constitution, the stability of the aromatic compound can be more improved.

## Claims

1. A stability improver of an aromatic compound comprising (A) at least one selected from resorcin and its derivatives, and salts thereof.

2. The stability improver of an aromatic compound according to claim 1, wherein the aromatic compound is at least one selected from an aromatic amine, an aromatic nitro compound, a phenol compound, an aromatic azo compound, and a fused ring compound.

3. The stability improver of an aromatic compound according to claim 1, wherein the aromatic compound is at least one selected from compounds represented by the following general formulae (1) to (3): 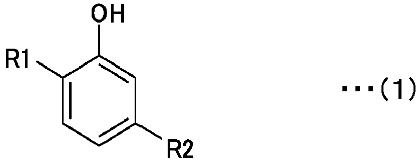 wherein
R1 represents a hydrogen atom or a methyl group; and R2 represents an amino group or a substituted amino group, 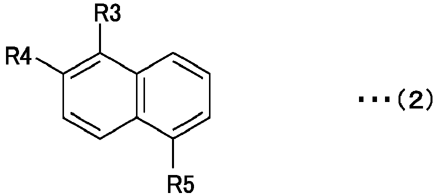 wherein
each of R3 to R5 represents a hydrogen atom or a hydroxyl group, and at least one of R3 to R5 represents a hydroxyl group, and 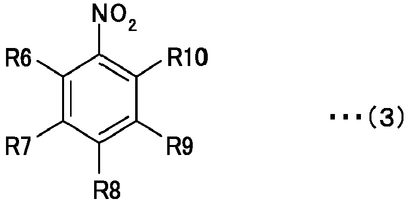 wherein
each of R6 to R10 represents a hydrogen atom, an amino group, a substituted amino group, a nitro group, or a hydroxyl group.

4. The stability improver of an aromatic compound according to claim 1, wherein the aromatic compound is at least one selected from m-aminophenol, 5-amino-o-cresol, α-naphthol, 5-(2-hydroxyethylamino)-2-methylphenol, 1,5-dihydroxynaphthalene, and a nitro dye.

5. A method for improving stability of an aromatic compound, which comprises mixing an aromatic compound with at least one compound selected from resorcin and its derivatives, and salts thereof in a solution.
